(19) Europäisches Patentamt / European Patent Office / Office européen des brevets

(11) EP 2 046 400 B1

(12) # EUROPÄISCHE PATENTSCHRIFT

(45) Veröffentlichungstag und Bekanntmachung des Hinweises auf die Patenterteilung:
**09.09.2015 Patentblatt 2015/37**

(21) Anmeldenummer: **07787228.1**

(22) Anmeldetag: **09.07.2007**

(51) Int Cl.:
***C08F 2/00*** (2006.01) ***A61L 15/22*** (2006.01)
***A61L 15/60*** (2006.01) ***C08F 2/10*** (2006.01)
***C08F 2/34*** (2006.01) ***C08F 20/04*** (2006.01)
***C08J 3/24*** (2006.01) ***C08F 220/06*** (2006.01)
*C08F 222/10* (2006.01)

(86) Internationale Anmeldenummer:
**PCT/EP2007/056952**

(87) Internationale Veröffentlichungsnummer:
**WO 2008/009580 (24.01.2008 Gazette 2008/04)**

(54) **VERFAHREN ZUR HERSTELLUNG NACHVERNETZTER WASSERABSORBIERENDER POLYMERPARTIKEL MIT HOHER ABSORPTION DURCH POLYMERISATION VON TROPFEN EINER MONOMERLÖSUNG**

METHOD FOR PRODUCING POST-CURED WATER-ABSORBENT POLYMER PARTICLES WITH A HIGHER ABSORPTION BY POLYMERISING DROPLETS OF A MONOMER SOLUTION

PROCÉDÉ DE PRODUCTION DE PARTICULES POLYMÈRES ABSORBANT L'EAU POST-POLYMÉRISÉES PRÉSENTANT UNE ABSORPTION ÉLEVÉE GRÂCE À LA POLYMÉRISATION DE GOUTELLETTES D'UNE SOLUTION MONOMÈRE

(84) Benannte Vertragsstaaten:
**AT BE BG CH CY CZ DE DK EE ES FI FR GB GR HU IE IS IT LI LT LU LV MC MT NL PL PT RO SE SI SK TR**

(30) Priorität: **19.07.2006 EP 06117488**

(43) Veröffentlichungstag der Anmeldung:
**15.04.2009 Patentblatt 2009/16**

(73) Patentinhaber: **BASF SE**
**67056 Ludwigshafen (DE)**

(72) Erfinder:
- **STUEVEN, Uwe**
**65812 Bad Soden (DE)**
- **WEISMANTEL, Matthias**
**63637 Jossgrund (DE)**
- **HEIDE, Wilfried**
**67251 Freinsheim (DE)**
- **KRÜGER, Marco**
**68161 Mannheim (DE)**
- **SEIDL, Volker**
**68199 Mannheim (DE)**
- **BLEI, Stefan**
**68163 Mannheim (DE)**
- **LÖSCH, Dennis**
**67122 Altrip (DE)**
- **FUNK, Rüdiger**
**65527 Niedernhausen (DE)**
- **HILLEBRECHT, Annemarie**
**36100 Petersberg (DE)**

(56) Entgegenhaltungen:
**WO-A-2005/080479 WO-A-2006/042704**
**DE-A1- 10 239 074 DE-A1-102004 057 868**

Bemerkungen:
Die Akte enthält technische Angaben, die nach dem Eingang der Anmeldung eingereicht wurden und die nicht in dieser Patentschrift enthalten sind.

EP 2 046 400 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Erfindung betrifft ein Verfahren zur Herstellung nachvernetzter wasserabsorbierender Polymerpartikel mit hoher Absorption durch Polymerisation von Tropfen einer Monomerlösung in einer die Tropfen umgebenden Gasphase, wobei der Feststoffgehalt der Monomerlösung mindestens 35 Gew.-% beträgt und die Polymerpartikel einen mittleren Durchmesser von mindestens 150 μm aufweisen.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Die Eigenschaften der wasserabsorbierenden Polymere können über den Vernetzungsgrad eingestellt werden. Mit steigendem Vernetzungsgrad steigt die Gelfestigkeit und sinkt die Absorptionskapazität. Dies bedeutet, dass mit steigender Absorption unter Druck (AUL) die Zentrifugenretentionskapazität (CRC) abnimmt (zu sehr hohen Vernetzungsgraden nimmt auch die Absorption unter Druck wieder ab).

Zur Verbesserung der Anwendungseigenschaften, wie beispielsweise Flüssigkeitsweiterleitung im gequollenen Gelbett (SFC) in der Windel und Absorption unter Druck (AUL), werden wasserabsorbierende Polymerpartikel im allgemeinen nachvernetzt. Dadurch steigt nur der Vernetzungsgrad der Partikeloberfläche, wodurch die Absorption unter Druck (AUL) und die Zentrifugenretentionskapazität (CRC) zumindest teilweise entkoppelt werden können. Diese Nachvernetzung kann in wässriger Gelphase durchgeführt werden. Vorzugsweise werden aber gemahlene und abgesiebte Polymerpartikel (Grundpolymer) an der Oberfläche mit einem Nachvernetzer beschichtet, thermisch nachvernetzt und getrocknet. Dazu geeignete Vernetzer sind Verbindungen, die mindestens zwei Gruppen enthalten, die mit den Carboxylatgruppen des hydrophilen Polymeren kovalente Bindungen bilden können.

Durch Sprühpolymerisation konnten die Verfahrensschritte Polymerisation und Trocknung zusammengefasst werden. Zusätzlich konnte die Partikelgröße durch geeignete Verfahrensführung in gewissen Grenzen eingestellt werden.

Die Herstellung wasserabsorbierender Polymerpartikel durch Polymerisation von Tropfen einer Monomerlösung wird beispielsweise in EP-A 0 348 180, WO 96/40427, US 5,269,980, DE-A 103 14 466, DE-A 103 40 253 und DE-A 10 2004 024 437 sowie den älteren deutschen Anmeldungen mit den Aktenzeichen 10 2005 002 412.2 und 10 2006 001 596.7 beschrieben.

DE-A 10 2004 042 946, DE-A 10 2004 042 948 und DE-A 10 2004 042 955 sowie die ältere deutsche Anmeldung mit dem Aktenzeichen 10 2005 019 398.6 beschreiben die Herstellung von Verdickern durch Sprühpolymerisation.

WO 2005/080479 A1 und DE 10 2004 057 868 A1 offenbaren Verfahren zu Herstellung oberflächennachvernetzter Polymerpartikel.

DE 102 39 074 A1 lehrt Beschichtung wasserabsorbierender Polymerpartikel mit wasserunlöslichen anorganischen Salzen.

WO 2006/042704 A1 lehrt die Beschichtung wasserabsorbierender Polymerpartikel mit wasserunlöslichen Metallphosphaten.

WO 2006/079631 A1 offenbart ein Verfahren zur Sprühpolymerisation mit anschließender Trocknung der erhaltenen Polymerpartikel im Wirbelbett.

Aufgabe der vorliegenden Erfindung war die Bereitstellung eines Verfahrens zur Herstellung wasserabsorbierender Polymerpartikel mit einem verbesserten Eigenschaftsprofil, d. h. einer hohen Zentrifugenretentionskapazität (CRC), einer hohen Absorption unter Druck (AUL) und einer hohen Quellgeschwindigkeit (FSR).

Gelöst wurde die Aufgabe durch ein Verfahren zur Herstellung wasserabsorbierender Polymerpartikel durch Polymerisation von Tropfen einer Monomerlösung, enthaltend

a) mindestens ein ethylenisch ungesättigtes Monomer,
b) mindestens einen Vernetzer,
c) mindestens einen Initiator,
d) Wasser,

in einer die Tropfen umgebenden Gasphase, wobei die erhaltenen Polymerpartikel nachvernetzt werden, dadurch gekennzeichnet, dass der Feststoffgehalt der Monomerlösung mindestens 35 Gew.-% beträgt und die Polymerpartikel einem mittleren Durchmesser von mindestens 150 μm aufweisen.

Die gemäß dem erfindungsgemäßen Verfahren erhältlichen wasserabsorbierenden Polymerpartikel weisen eine Zentrifugenretentionskapazität (CRC) von typischerweise mindestens 20 g/g, vorzugsweise mindestens 25 g/g, bevorzugt mindestens 30 g/g, besonders bevorzugt mindestens 35 g/g, ganz besonders bevorzugt mindestens 40 g/g, auf. Die Zentrifugenretentionskapazität (CRC) der wasserabsorbierenden Polymerpartikel beträgt üblicherweise weniger als 60 g/g.

Die Absorption unter einem Druck von 4,83 kPa (AUL0.7psi) der gemäß dem erfindungsgemäßen Verfahren erhältlichen wasserabsorbierenden Polymerpartikel erfüllt die Bedingung

$$AUL0.7psi \geq a\,CRC^2 + b\,CRC + c\,.$$

Der Koeffizient a beträgt -0,017, der Koeffizient b beträgt +1,5 und der Koeffizient c beträgt typischerweise -6, vorzugsweise -5,5, besonders bevorzugt -5, ganz besonders bevorzugt -4,5. Die Absorption unter einem Druck von 4,83 kPa erfüllt üblicherweise die Bedingung

$$a\,CRC^2 + b\,CRC \geq AUL0.7psi \geq a\,CRC^2 + b\,CRC + c\,.$$

Der funktionale Zusammenhang gilt für Zentrifugenretentionskapazitäten (CRC) von ca. 20 g/g bis ca. 50 g/g.

Die Quellgeschwindigkeit (FSR) der gemäß dem erfindungsgemäßen Verfahren erhältlichen wasserabsorbierenden Polymerpartikel erfüllt die Bedingung

$$FSR \geq a\,CRC^2 + b\,CRC + c\,.$$

Der Koeffizient a beträgt -0,00082, der Koeffizient b beträgt +0,07 und der Koeffizient c beträgt typischerweise -0,95, vorzugsweise -0,9, besonders bevorzugt -0,85, ganz besonders bevorzugt -0,8. Die Quellgeschwindigkeit (FSR) erfüllt üblicherweise die Bedingung

$$a\,CRC^2 + b\,CRC - 0{,}5 \geq FSR \geq a\,CRC^2 + b\,CRC + c\,.$$

Der funktionale Zusammenhang gilt für Zentrifugenretentionskapazitäten (CRC) von ca. 20 g/g bis ca. 50 g/g.

Die gemäß dem erfindungsgemäßen Verfahren erhältlichen wasserabsorbierenden Polymerpartikel weisen eine Permeabilität (SFC) von typischerweise mindestens 2 x $10^{-7}$ $cm^3s/g$, vorzugsweise mindestens 10 x $10^{-7}$ $cm^3s/g$, bevorzugt mindestens 30 x $10^{-7}$ $cm^3s/g$, besonders bevorzugt mindestens 60 x $10^{-7}$ $cm^3s/g$, ganz besonders bevorzugt mindestens 200 x $10^{-7}$ $cm^3s/g$, auf. Die Permeabilität (SFC) der wasserabsorbierenden Polymerpartikel beträgt üblicherweise weniger als 500 x $10^{-7}$ $cm^3s/g$.

Der Feststoffgehalt der Monomerlösung beträgt vorzugsweise mindestens 38 Gew.-%, bevorzugt mindestens 40 Gew.-%, besonders bevorzugt mindestens 41 Gew.-%, ganz besonders bevorzugt mindestens 42 Gew.-%. Dabei ist der Feststoffgehalt die Summe aller nach der Polymerisation nichtflüchtigen Bestandteile. Dies sind Monomer a), Vernetzer b) und Initiator c).

Das Eigenschaftsprofil der wasserabsorbierenden Polymerpartikel verbessert sich mit steigendem Feststoffgehalt. Der Feststoffgehalt wird nur durch die Löslichkeit nach oben begrenzt.

Der mittlere Durchmesser der Polymerpartikel beträgt vorzugsweise mindestens 200 μm, besonders bevorzugt von 250 bis 600 μm, ganz besonders von 300 bis 500 μm, wobei der Partikeldurchmesser durch Lichtstreuung bestimmt wird und den volumengemittelten mittleren Durchmesser bedeutet. 90% der Polymerpartikel weisen einen Durchmesser von vorzugsweise 100 bis 800 μm, besonders bevorzugt von 150 bis 700 μm, ganz besonders bevorzugt von 200 bis 600 μm, auf.

Der Sauerstoffgehalt der Gasphase beträgt vorzugsweise 0,001 bis 0,15 Vol.-%, besonders bevorzugt 0,002 bis 0,1 Vol.-%, ganz besonders bevorzugt 0,005 bis 0,05 Vol.-%.

Die Gasphase enthält neben Sauerstoff vorzugsweise nur inerte Gase, d.h. Gase, die unter Reaktionsbedingungen nicht in die Polymerisation eingreifen, beispielsweise Stickstoff und/oder Wasserdampf.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser, und haben vorzugsweise mindestens je eine Säuregruppe.

Geeignete Monomere a) sind beispielsweise ethylenisch ungesättigte Carbonsäuren, wie Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die bevorzugten Monomere a) haben mindestens eine Säuregruppe, wobei die Säuregruppen vorzugsweise zumindest teilweise neutralisiert sind.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) sind üblicherweise teilweise neutralisiert, vorzugsweise zu 25 bis 85 mol-%, bevorzugt zu 50 bis 80 mol-%, besonders bevorzugt 60 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ) und/oder Tocopherole.

Unter Tocopherol werden Verbindungen der folgenden Formel verstanden

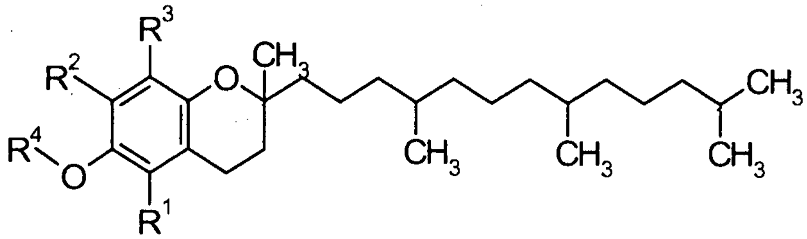

wobei $R^1$ Wasserstoff oder Methyl, $R^2$ Wasserstoff oder Methyl, $R^3$ Wasserstoff oder Methyl und $R^4$ Wasserstoff oder ein Säurerest mit 1 bis 20 Kohlenstoffatomen bedeutet.

Bevorzugte Reste für $R^4$ sind Acetyl, Ascorbyl, Succinyl, Nicotinyl und andere physiologisch verträgliche Carbonsäuren. Die Carbonsäuren können Mono-, Di- oder Tricarbonsäuren sein.

Bevorzugt ist alpha-Tocopherol mit $R^1 = R^2 = R^3$ = Methyl, insbesondere racemisches alpha-Tocopherol. $R^1$ ist besonders bevorzugt Wasserstoff oder Acetyl. Insbesondere bevorzugt ist RRR-alpha-Tocopherol.

Die Monomerlösung enthält bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Vernetzer b) sind Verbindungen mit mindestens zwei radikalisch polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP-A 0 530 438 beschrieben, Di- und Triacrylate, wie in EP-A 0 547 847,

EP-A 0 559 476, EP-A 0 632 068, WO 93/21237, WO 03/104299, WO 03/104300, WO 03/104301 und in DE-A 103 31 450 beschrieben, gemischte Acrylate, die neben Acrylatgruppen weitere ethylenisch ungesättigte Gruppen enthalten, wie in DE-A 103 314 56 und DE-A 103 55 401 beschrieben, oder Vernetzermischungen, wie beispielsweise in DE-A 195 43 368, DE-A 196 46 484, WO 90/15830 und WO 02/32962 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP-A 0 343 427 beschrieben sind. Weiterhin geeignete Vernetzer b) sind Pentaerythritoldi- Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1.000 aufweist.

Besonders vorteilhafte Vernetzer b) sind jedoch Di- und Triacrylate des 3- bis 15-fach ethoxylierten Glyzerins, des 3- bis 15-fäch ethoxylierten Trimethylolpropans, des 3- bis 15-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine wie sie beispielsweise in WO 03/104301 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5- fach ethoxylierten und/oder propoxylierten Glyzerins. Am meisten bevorzugt sind die Triacrylate des 3- bis 5-fach ethoxylierten und/oder propoxylierten Glyzerins. Diese zeichnen sich durch besonders niedrige Restgehalte (typischerweise unter 10 Gew.-ppm) im wasserabsorbierenden Polymer aus und die wässrigen Extrakte der damit hergestellten wasserabsorbierenden Polymere weisen eine fast unveränderte Oberflächenspannung (typischerweise mindestens 0,068 N/m) im Vergleich zu Wasser gleicher Temperatur auf.

Die Monomerlösung enthält typischerweise mindestens 0,4 Gew.-%, vorzugsweise mindestens 0,6 Gew.-%, bevorzugt mindestens 0,8 Gew.-%, besonders bevorzugt mindestens 1,5 Gew.-%, ganz besonders bevorzugt mindestens 3,0 Gew.-%, Vernetzer b), jeweils bezogen auf Monomer a).

Als Initiatoren c) können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die sogenannten Redoxinitiatoren. Bevorzugt ist der Einsatz von wasserlöslichen Initiatoren. In manchen Fällen ist es vorteilhaft, Mischungen verschiedener Initiatoren zu verwenden, beispielsweise Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden.

Besonders bevorzugte Initiatoren c) sind Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/ Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Die Initiatoren werden in üblichen Mengen eingesetzt, beispielsweise in Mengen von 0,001 bis 5 Gew.-%, vorzugsweise 0,01 bis 1 Gew.-%, bezogen auf die Monomeren a).

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, gesenkt.

Die Polymerisationsinhibitoren können auch durch Absorption, beispielsweise an Aktivkohle, entfernt werden.

Die Monomerlösung wird zur Polymerisation in der Gasphase vertropft.

Die Polymerisation in den Monomerlösungstropfen findet vorzugsweise in homogener Phase statt. Dies bedeutet, dass die Monomerlösung homogen ist und dass die Monomerlösung auch während der Polymerisation homogen bleibt. Das Polymer darf während der Polymerisation quellen, sollte aber nicht ausfallen und eine zweite Phase im Tropfen bilden. Ansonsten würden in jedem Tropfen mehrere Polymerkeime entstehen, die während der Trocknung Agglomerate sehr kleiner Primärpartikel bilden. Ziel des erfindungsgemäßen Verfahrens ist vorzugsweise die Herstellung jeweils eines Primärpartikels pro Tropfen. Daher sollten die Monomeren a) und die Vernetzer b) so ausgewählt werden, dass das entstehende Polymer in der wäßrigen Phase des Tropfens quellbar ist.

Daher wird das erfindungsgemäße Verfahren vorzugsweise in Abwesenheit hydrophober inerter Lösungsmittel durchgeführt. Hydrophobe, inerte Lösungsmittel sind praktisch alle mit Wasser nicht mischbaren Flüssigkeiten, die nicht in die Polymerisation eingreifen, d.h. keine polymerisierbaren Gruppen enthalten. Mit Wasser nicht mischbar bedeutet, dass die Löslichkeit der hydrophoben Lösungsmittel in Wasser weniger als 5 g/100 g, vorzugsweise weniger als 1 g/100 g, besonders bevorzugt weniger als 0,5 g/100 g, beträgt.

Bei der Vertropfung wird eine Monomerlösung unter Ausbildung von Tropfen in die Gasphase dosiert. Die Vertropfung der Monomerlösung kann beispielsweise mittels einer Vertropferplatte durchgeführt werden.

Eine Vertropferplatte ist eine Platte mit mindestens einer Bohrung, wobei die Flüssigkeit von oben durch die Bohrung tritt. Die Vertropferplatte bzw. die Flüssigkeit kann in Schwingungen versetzt werden, wodurch an der Unterseite der Vertropferplatte je Bohrung eine idealerweise monodisperse Tropfenkette erzeugt wird.

Die Anzahl und die Größe der Bohrungen werden gemäß der gewünschten Kapazität und Tropfengröße ausgewählt. Der Tropfendurchmesser beträgt dabei üblicherweise das 1,9fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Der Vertropfer wird im Strömungsbereich des laminaren Strahlzerfalls betrieben, d.h. die Reynoldszahl bezogen auf den Durchsatz pro Bohrung und den Bohrungsdurchmesser ist vorzugsweise kleiner als 2.000, bevorzugt kleiner 1.000, besonders bevorzugt kleiner 500, ganz besonders bevorzugt kleiner

100. Der Druckverlust über die Bohrung beträgt vorzugsweise weniger als 2,5 bar, besonders bevorzugt weniger als 1,5 bar, ganz besonders bevorzugt weniger als 1 bar.

Die Vertropferplatte weist üblicherweise mindestens eine, vorzugsweise mindestens 10, besonders bevorzugt mindestens 50, und üblicherweise bis zu 10.000, vorzugsweise bis zu 5.000, besonders bevorzugt bis zu 1.000, Bohrungen auf, wobei die Bohrungen üblicherweise gleichmäßig über die Vertropferplatte verteilt sind, vorzugsweise in der sogenannten Dreiecksteilung, d.h. jeweils drei Bohrungen bilden die Ecken eines gleichseitigen Dreiecks.

Der Durchmesser der Bohrungen wird an die gewünschte Tropfengröße angepasst.

Es kann vorteilhaft sein die Vertropferplatte auf eine Trägerplatte aufzulegen, wobei die Trägerplatte ebenfalls Bohrungen aufweist. Dabei weisen die Bohrungen der Trägerplatte einen größeren Durchmesser auf als die Bohrungen der Vertropferplatte auf und sind so angeordnet, dass sich unter jeder Bohrung der Vertropferplatte eine mit ihr konzentrische Bohrung der Trägerplatte befindet. Diese Anordnung ermöglicht einen schnellen Wechsel der Vertropferplatte, beispielsweise um Tropfen einer anderen Größe zu erzeugen.

Die Vertropfung kann aber auch mittels pneumatischer Ziehdüsen, Rotation, Zerschneiden eines Strahls oder schnell ansteuerbarer Mikroventildüsen durchgeführt werden.

In einer pneumatischen Ziehdüse wird ein Flüssigkeitsstrahl zusammen mit einem Gasstrom durch eine Blende beschleunigt. Über die Gasmenge kann der Durchmesser des Flüssigkeitsstrahls und damit der Tropfendurchmesser beeinflusst werden.

Bei der Vertropfung durch Rotation tritt die Flüssigkeit durch die Öffnungen einer rotierenden Scheibe. Durch die auf die Flüssigkeit wirkende Fliehkraft werden Tropfen definierter Größe abgerissen. Die Rotationsvertropfung wird beispielsweise in DE-A 4308842 und US 6338438 beschrieben.

Der austretende Flüssigkeitsstrahl kann aber auch mittels eines rotierenden Messers in definierte Segmente zerschnitten werden. Jedes Segment bildet anschließend einen Tropfen.

Bei Verwendung von Mikroventildüsen werden direkt Tropfen mit definiertem Flüssigkeitsvolumen erzeugt.

Bevorzugt strömt die Gasphase als Trägergas durch den Reaktionsraum. Dabei kann das Trägergas im Gleichstrom oder im Gegenstrom zu den frei fallenden Tropfen der Monomerlösung durch den Reaktionsraum geführt werden, bevorzugt im Gleichstrom. Vorzugsweise wird das Trägergas nach einem Durchgang zumindest teilweise, bevorzugt zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1%.

Die Polymerisation wird vorzugsweise in einer laminaren Gasströmung durchgeführt. Eine laminare Gasströmung ist eine Gasströmung, bei der sich die einzelnen Schichten der Strömung nicht vermischen, sondern parallel bewegen. Ein Maß für die Strömungsverhältnisse ist die Reynolds-Zahl (Re). Unterhalb einer kritischen Reynolds-Zahl ($Re_{krit}$) von 2300 ist die Gasströmung laminar. Die Reynolds-Zahl der laminaren Gasströmung beträgt vorzugsweise weniger als 2000, besonders bevorzugt weniger als 1500, ganz besonders bevorzugt weniger als 1000. Der untere Grenzfall der laminaren Inertgasströmung ist eine ruhende Inertgasatmosphäre (Re = 0), d.h., es wird nicht kontinuierlich Inertgas eingespeist.

Die Gasgeschwindigkeit wird vorzugsweise so eingestellt, dass die Strömung im Reaktor gerichtet ist, beispielsweise liegen keine der allgemeinen Strömungsrichtung entgegengesetzte Konvektionswirbel vor, und beträgt beispielsweise 0,1 bis 2 m/s, vorzugsweise 0,5 bis 1,8 m/s, bevorzugt 1 bis 1,5 m/s.

Das Trägergas wird zweckmäßigerweise vor dem Reaktor auf die Reaktionstemperatur vorgewärmt.

Die Reaktionstemperatur beträgt bei der thermisch induzierten Polymerisation vorzugsweise 70 bis 250°C, besonders bevorzugt 100 bis 220°C, ganz besonders bevorzugt 120 bis 200°C.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das den Reaktionsraum verlassende Trägergas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Trägergas ersetzt werden, wobei im Reaktionsabgas enthaltenes Wasser und nicht umgesetzte Monomere a) abgetrennt und rückgeführt werden können.

Besonders bevorzugt ist ein Wärmeverbund, dass heißt, ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Das Reaktionsprodukt kann dem Reaktor in üblicher Weise entnommen werden, beispielsweise am Boden über eine Förderschnecke, und gegebenenfalls bis zur gewünschten Restfeuchte und zum gewünschten Restmonomerengehalt getrocknet werden.

Die Polymerpartikel werden anschließend zur weiteren Verbesserung der Eigenschaften nachvernetzt.

Geeignete Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyglycidylverbindungen, wie in EP-A 0 083 022, EP-A 0 543 303 und EP-A 0 937 736 beschrieben, di- oder polyfunktionelle Alkohole, wie in DE-C-33 14 019, DE-C-35 23 617 und EP-A 0 450 922 beschrieben, oder ß-Hydroxyalkylamide, wie in DE-A 102 04 938 und US 6,239,230 beschrieben.

Des weiteren sind in DE-A 40 20 780 zyklische Karbonate, in DE-A 198 07 502 2-Oxazolidon und dessen Derivate, wie 2-Hydroxyethyl-2-oxazolidon, in DE-A 198 07 992 Bis- und Poly-2-oxazolidinone, in DE-A 198 54 573 2-Oxotetrahydro-1,3-oxazin und dessen Derivate, in DE-A 198 54 574 N-Acyl-2-Oxazolidone, in DE-A 102 04 937 zyklische Harnstoffe, in DE-A 103 34 584 bizyklische Amidacetale, in EP-A 1 199 327 Oxetane und zyklische Harnstoffe und in WO 03/031482 Morpholin-2,3-dion und dessen Derivate als geeignete Nachvernetzer beschrieben.

Die Menge an Nachvernetzer beträgt vorzugsweise 0,01 bis 1 Gew.-%, besonders bevorzugt 0,05 bis 0,5 Gew.-%, ganz besonders bevorzugt 0,1 bis 0,2 Gew.-%, jeweils bezogen auf das Polymer.

Die Nachvernetzung wird üblicherweise so durchgeführt, dass eine Lösung des Nachvernetzers auf das Hydrogel oder die trockenen Polymerpartikel aufgesprüht wird. Im Anschluss an das Aufsprühen wird thermisch getrocknet, wobei die Nachvernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige®-Mischer, Bepex®-Mischer, Nauta®-Mischer, Processall®-Mischer und Schugi®-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex®-Trockner und Nara®-Trockner. Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 170 bis 250°C, bevorzugt 180 bis 220°C, und besonders bevorzugt 190 bis 210°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten.

Das erfindungsgemäße Verfahren ermöglicht die Herstellung wasserabsorbierender Polymerpartikel mit einer hohen Zentrifugenretentionskapazität (CRC) und einer hohen Absorption unter einem Druck von 4,83 kPa (AUL0.7psi).

Die erfindungsgemäßen wasserabsorbierenden Polymerpartikel weisen einen Gehalt an hydrophobem Lösungsmittel von typischerweise weniger als 0,005 Gew.-%, vorzugsweise weniger als 0,002 Gew.-%, besonders bevorzugt weniger als 0,001 Gew.-%, ganz besonders bevorzugt weniger als 0,0005 Gew.-%, auf. Der Gehalt an hydrophobem Lösungsmittel wird gaschromatographisch mittels der Head-Space-Technik bestimmt.

Polymerpartikel, die durch umgekehrte Suspensionspolymerisation erhalten wurden, enthalten üblicherweise noch ca. 0,01 Gew.-% des als Reaktionsmediums verwendeten hydrophoben Lösungsmittels.

Die erfindungsgemäßen wasserabsorbierenden Polymerpartikel weisen einen Tensidgehalt von typischerweise weniger als 1 Gew.-%, vorzugsweise weniger als 0,5 Gew.-%, besonders bevorzugt weniger als 0,1 Gew.-%, ganz besonders bevorzugt weniger als 0,05 Gew.-%, auf.

Polymerpartikel, die durch umgekehrte Suspensionspolymerisation erhalten wurden, enthalten üblicherweise noch mindestens 1 Gew.-% des zur Stabilisierung der Suspension verwendeten Tensids.

Die erfindungsgemäßen wasserabsorbierenden Polymerpartikel sind annähernd rund, d.h. die Polymerpartikel weisen eine mittlere Sphärizität von typischerweise mindestens 0,84, vorzugsweise mindestens 0,86, besonders bevorzugt mindestens 0,88, ganz besonders bevorzugt mindestens 0,9, auf. Die Sphärizität (SPHT) ist definiert als

$$SPHT = \frac{4\pi A}{U^2},$$

wobei A die Querschnittsfläche und U der Querschnittsumfang der Polymerpartikel ist. Die mittlere Sphärizität ist die volumengemittelte Sphärizität.

Die mittlere Sphärizität kann beispielsweise mit dem Bildanalysesystem Camsizer® (Retsch Technolgy GmbH; DE) bestimmt werden:

Zur Messung wird das Produkt über einen Trichter aufgegeben und mit einer Dosierrinne zum Fallschacht gefördert.

Während die Partikel an einer Leuchtwand vorbeifallen werden sie wahlweise von einer Kamera erfasst. Die aufgenommenen Bilder werden von der Software entsprechend der ausgewählten Parameter ausgewertet.

Zur Charakterisierung der Rundheit wird die im Programm mit Sphärizität gekennzeichnete Messgröße herangezogen. Angegeben sind die mittleren, mit dem Volumen gewichteten Sphärizitäten, wobei das Volumen der Partikel über den Äquivalentdurchmesser $xc_{min}$ ermittelt werden. Zur Bestimmung des Äquivalentdurchmessers $xc_{min}$ wird der jeweils längste Sehnendurchmesser für insgesamt 32 unterschiedliche Raumrichtungen gemessen. Der Äquivalentdurchmesser $xc_{min}$ ist der kürzeste dieser 32 Sehnendurchmesser. Der Äquivalentdurchmesser $xc_{min}$ entspricht der Maschenweite eines Siebes, das das Partikel gerade noch passieren kann. Zur Erfassung der Partikel wird die sogenannte CCD-Zoom Kamera (CAM-Z) eingesetzt. Zur Steuerung der Dosierrinne wird ein Flächenbelegungsanteil von 0,5% vorgegeben.

Polymerpartikel mit relativ niedriger Sphärizität werden durch umgekehrte Suspensionspolymerisation erhalten, wenn die Polymerpartikel während oder nach der Polymerisation agglomeriert werden.

Die durch übliche Lösungspolymerisation (Gelpolymerisation) hergestellten wasserabsorbierende Polymerpartikel werden nach der Trocknung gemahlen und klassiert wobei unregelmäßige Polymerpartikel erhalten werden. Die mittlere Sphärizität dieser Polymerpartikel beträgt zwischen ca. 0,72 und ca. 0,78.

Ein weiterer Gegenstand der vorliegenden Erfindung sind Verfahren zur Herstellung von Hygieneartikeln, insbesondere Windeln, umfassend die Verwendung gemäß obengenannten Verfahrens hergestellter wasserabsorbierender Polymerpartikel.

Ein weiterer Gegenstand der vorliegenden Erfindung ist die Verwendung erfindungsgemäßer wasserabsorbierender Polymerpartikel in Hygieneartikeln, zur Verdickung von Abfällen, insbesondere medizinischen Abfällen, oder als wasserrückhaltendes Mittel in der Landwirtschaft.

Die wasserabsorbierenden Polymerpartikel werden mittels der nachfolgend beschriebenen Testmethoden geprüft.

Methoden:

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

Flüssigkeitsweiterleitung (SFC Saline Flow Conductivity)

Die Flüssigkeitsweiterleitung einer gequollenen Gelschicht unter Druckbelastung von 0,3 psi (2070 Pa) wird, wie in EP-A 0 640 330 beschrieben, als Gel-Layer-Permeability einer gequollenen Gelschicht aus superabsorbierendem Polymer bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP-A 0 640 330. Der Durchfluss wird automatisch erfasst.

Die Flüssigkeitsweiterleitung (SFC) wird wie folgt berechnet:

$$SFC\ [cm^3s/g] = (Fg(t=0) x L0)/(d x A x WP),$$

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in $g/cm^3$, A die Fläche der Gelschicht in $cm^2$ und WP der hydrostatische Druck über der Gelschicht in $dyn/cm^2$ darstellt.

Quellgeschwindigkeit (FSR Free Swell Rate)

Zur Bestimmung der Quellgeschwindigkeit werden 1,00 g (= $W_1$) der trockenen wasserabsorbierenden Polymerpartikel in ein 25 ml Becherglas eingewogen und gleichmässig auf dessen Boden verteilt. Dann wurden 20 ml einer 0,9 gew.-%igen Kochsalzlösung mittels in ein zweites Becherglas dosiert und der Inhalt dieses Glases wurde dem ersten zügig hinzugefügt und eine Stopuhr gestartet. Sobald der letzte Tropfen Salzlösung absorbiert wurde, was man am Verschwinden der Reflexion auf der Flüssigkeitsoberfläche erkennt, wurde die Stopuhr angehalten. Die genaue Flüssigkeitsmenge, die aus dem zweiten Becherglas ausgegossen und durch das Polymer im ersten Becherglas absorbiert wurde, wurde durch Rückwägung des zweiten Becherglases genau bestimmt (= $W_2$). Die für die Absorption benötigte Zeitspanne, die mit der Stopuhr gemessen wurde, wurde als t bezeichnet. Das Verschwinden des letzten Flüssigkeitstropfens auf der Oberfläche wurde als Zeitpunkt t bestimmt.

Daraus errechnet sich die Quellgeschwindigkeit (FSR) wie folgt:

$$FSR\ [g/gs] = W_2/(W_1 x t)$$

Zentrifugenretentionskapazität (CRC Centrifuge Retention Capacity)

Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

Absorption unter Druck (AUL0.7psi Absorbency Under Load)

Die Absorption unter Druck wird gemäß der von der EDANA (European Disposables and Nonwovens Association) empfohlenen Testmethode Nr. 442.2-02 " Absorption under pressure" bestimmt.

Die EDANA-Testmethoden sind beispielsweise erhältlich bei der European Disposables and Nonwovens Association, Avenue Eugene Plasky 157, B-1030 Brüssel, Belgien.

Beispiele

Beispiele 1 bis 7

14,6 kg Natriumacrylat (37,5 gew.-%ige Lösung in Wasser) und 1,4 kg Acrylsäure wurden mit 11,2 g bis 168 g 15-fach ethoxiliertem Trimethylolpropantriacrylat gemischt. Die Lösung wurde in einen erwärmten, mit Stickstoffatmosphäre gefüllten Vertropfungsturm vertropft (180°C, 12m Höhe, 2m Breite, Gasgeschwindigkeit 0,1 m/s im Gleichstrom). Die Dosiergeschwindigkeit betrug 16 kg/h. Die Vertropferplatte wies 37 Bohrungen à 170 µm auf. Der Durchmesser der Vertropferplatte betrug 65 mm. Der Initiator wurde kurz vor dem Vertropfer über einen Venturi-Mischer in die Monomerlösung dosiert. Als Initiator wurde eine 15 gew.-%ige Lösung von 2,2'-Azobis[2-(2-imidazolin-2-yl)propan]dihydrochlorid in Wasser verwendet. Die Dosiergeschwindigkeit der Initiatorlösung betrug 0,224 kg/h. Die Gasaustrittstemperatur aus dem Vertropfungsturm betrug 130°C. Der mittlere Teilchendurchmesser der erhaltenen Polymerpartikel betrug 270 µm.

Anschließend wurden die erhaltenen wasserabsorbierenden Polymerpartikel nachvernetzt. Dazu wurden 20 g wasserabsorbierende Polymerpartikel in einem Waring®-Labormischer auf mittlerer Rührstufe mittels einer Injektionsspritze mit einer Nachvernetzerlösung besprüht. Das feuchte Polymer wurde mit einem Spatel nochmals homogenisiert, in einer Petrischale mit einem Innendurchmesser von 18,5 cm gleichmäßig verteilt und in einem Umlufttrockenschrank wärmebehandelt. Die nachvernetzten Polymerpartikel wurden über ein 600 µm Sieb von Klumpen befreit und analysiert.

Nachvernetzung A: Die Nachvernetzerlösung bestand aus 0,016 g Diethylenglykoldiglycidylether, 0,35 g Wasser und 0,234 g Propylenglykol. Das feuchte Polymer wurde 30 Minuten bei 120°C im Umlufttrockenschrank wärmebehandelt.

Nachvernetzung B: Die Nachvernetzerlösung bestand aus 0,03 g N-Hydroxyethyloxazolidin-2-on, 0,42 g Wasser und 0,18 g Isopropanol. Das feuchte Polymer wurde 60 Minuten bei 170°C im Umlufttrockenschrank wärmebehandelt.

Die Ergebnisse sind in den Tabellen 1 und 2 zusammengefasst:

Tab. 1: Nachvernetzung mit Diethylenglykoldiglycidylether (Nachvernetzung A)

| Bsp. | Vernetzergehalt*) | CRC [g/g] | AUL0.7psi [g/g] | SFC [$10^{-7}cm^3s/g$] | FSR [g/gs] |
|---|---|---|---|---|---|
| 1 | 0,2 Gew.-% | 41,9 | 28,3 | 2 | 0,75 |
| 2 | 0,3 Gew.-% | 37,2 | 28,1 | 10 | 0,59 |
| 3 | 0,4 Gew.-% | 34,8 | 27,2 | 16 | 0,58 |
| 4 | 0,6 Gew.-% | 31,2 | 25,6 | 36 | 0,65 |
| 5 | 0,8 Gew.-% | 28,7 | 24,5 | 51 | 0,51 |
| 6 | 1,5 Gew.-% | 26,4 | 22,3 | 76 | 0,53 |

(fortgesetzt)

| Bsp. | Vernetzergehalt*) | CRC [g/g] | AUL0.7psi [g/g] | SFC [$10^{-7}cm^3s/g$] | FSR [g/gs] |
|---|---|---|---|---|---|
| 7 | 3,0 Gew.-% | 23,1 | 21,4 | 203 | 0,30 |
| *) bezogen auf Acrylsäure | | | | | |

Tab. 1 (Fortsetzung):

| Bsp. | AUL0.7psi [g/g] | -0,017 $CRC^2$+1,5 CRC-6 [g/g] | FSR [g/gs] | -0,00082 $CRC^2$+0,07 CRC-0,95 [g/gs] |
|---|---|---|---|---|
| 1 | 28,3 | 27,0 | 0,75 | 0,54 |
| 2 | 28,1 | 26,3 | 0,59 | 0,52 |
| 3 | 27,2 | 25,6 | 0,58 | 0,49 |
| 4 | 25,6 | 24,3 | 0,65 | 0,42 |
| 5 | 24,5 | 23,0 | 0,51 | 0,38 |
| 6 | 22,3 | 21,8 | 0,53 | 0,33 |
| 7 | 21,4 | 19,6 | 0,30 | 0,23 |

Tab. 2: Nachvernetzung mit N-Hydroxyethyl-oxazolidin-2-on (Nachvernetzung B)

| Bsp. | Vernetzergehalt*) | CRC [g/g] | AUL0.7psi [g/g] | SFC [$10^{-7}$ $cm^3s/g$] | FSR [g/gs] |
|---|---|---|---|---|---|
| 1 | 0,2 Gew.-% | 41,7 | 28,1 | 3 | 0,63 |
| 2 | 0,3 Gew.-% | 39,6 | 29,0 | 3 | 0,70 |
| 3 | 0,4 Gew.-% | 35,7 | 29,1 | 10 | 0,59 |
| 4 | 0,6 Gew.-% | 32,6 | 25,0 | 25 | 0,61 |
| 5 | 0,8 Gew.-% | 30,2 | 27,1 | 37 | 0,42 |
| 6 | 1,5 Gew.-% | 27,7 | 23,4 | 65 | 0,60 |
| 7 | 3,0 Gew.-% | 22,9 | 21,6 | 216 | 0,31 |
| *) bezogen auf Acrylsäure | | | | | |

Tab. 2 (Fortsetzung):

| Bsp. | AUL0.7psi [g/g] | -0,017 $CRC^2$+1,5 CRC-6 [g/g] | FSR [g/gs] | -0,00082 $CRC^2$+0,07 CRC-0,95 [g/gs] |
|---|---|---|---|---|
| 1 | 28,1 | 27,0 | 0,63 | 0,54 |
| 2 | 29,0 | 26,7 | 0,70 | 0,54 |
| 3 | 29,1 | 25,9 | 0,59 | 0,50 |
| 4 | 25,0 | 24,8 | 0,61 | 0,46 |
| 5 | 27,1 | 23,8 | 0,42 | 0,42 |
| 6 | 23,4 | 22,5 | 0,60 | 0,36 |
| 7 | 21,6 | 19,4 | 0,31 | 0,22 |

Beispiel 8

14,6 kg Kaliumacrylat (45,0 gew.-%ige Lösung in Wasser) und 1,4 kg Acrylsäure wurden mit 17,1 g 15-fach ethoxiliertem Trimethylolpropantriacrylat gemischt. Die Lösung wurde in einen erwärmten, mit Stickstoffatmosphäre

gefüllten Vertropfungsturm vertropft (180°C, 12m Höhe, 2m Breite, Gasgeschwindigkeit 0,1 m/s im Gleichstrom). Die Dosiergeschwindigkeit betrug 16 kg/h. Die Vertropferplatte wies 37 Bohrungen à 170 $\mu$m auf. Der Durchmesser der Vertropferplatte betrug 65 mm. Der Initiator wurde kurz vor dem Vertropfer über einen Venturi-Mischer in die Monomerlösung dosiert. Als Initiator wurde eine 15 gew.-%ige Lösung von 2,2'-Azobis[2-(2-imidazolin-2-yl)propan]dihydrochlorid in Wasser verwendet. Die Dosiergeschwindigkeit der Initiatorlösung betrug 0,224 kg/h. Die Gasaustrittstemperatur aus dem Vertropfungsturm betrug 130°C. Der mittlere Teilchendurchmesser der erhaltenen Polymerpartikel betrug 270 $\mu$m. Anschließend wurden die erhaltenen wasserabsorbierenden Polymerpartikel nachvernetzt.

Nachvernetzung A: Die erhaltenen Polymerpartikel hatten eine CRC von 35,1 g/g, eine AUL0.7psi von 27,6 g/g und eine SFC von 17 x $1{,}0^{-7}$ $cm^3$g/s. Der Wert für -0,017 $CRC^2$ + 1,5 CRC - 6 betrug 25,7 g/g.

Nachvernetzung B: Die erhaltenen Polymerpartikel hatten eine CRC von 36,0 g/g, eine AUL0.7psi von 29,5 g/g und eine SFC von 11 x $10^{-7}$ $cm^3$g/s. Der Wert für -0,017 $CRC^2$ + 1,5 CRC - 6 betrug 26,0 g/g.

Vergleichsbeispiel:

Zum Vergleich wurden handelsübliche nachvernetzte wasserabsorbierende Polymerpartikel (Hysorb® M 7055, BASF Aktiengesellschaft, DE) untersucht.

Die Polymerpartikel hatten eine CRC von 31,1 g/g, eine AUL0.7psi von 22,1 g/g, eine SFC von 31 x $10^{-7}$ $cm^3$g/s und eine FSR von 0,24 g/gs. Der Wert für -0,017 $CRC^2$ + 1,5 CRC - 6 betrug 24,2 g/g. Der Wert für -0,00082 $CRC^2$ + 0,07 CRC - 0,95 betrug 0,43 g/gs.

## Patentansprüche

**1.** Verfahren zur Herstellung wasserabsorbierender Polymerpartikel durch Polymerisation von Tropfen einer Monomerlösung, enthaltend

a) mindestens ein ethylenisch ungesättigtes Monomer,
b) mindestens einen Vernetzer,
c) mindestens einen Initiator,
d) Wasser,

in einer die Tropfen umgebenden Gasphase, wobei die erhaltenen Polymerpartikel nachvernetzt werden, **dadurch gekennzeichnet, dass** der Feststoffgehalt der Monomerlösung mindestens 35 Gew.-% beträgt und die Polymerpartikel einen mittleren Durchmesser von mindestens 150 $\mu$m aufweisen, wobei, der Feststoffgehalt die Summe aus Monomer a), Vernetzer b) und Initiator c) ist, der mittlere Partikeldurchmesser durch Lichtstreuung bestimmt wird und den volumengemittelten mittleren Durchmesser bedeutet.

**2.** Verfahren gemäß Anspruch 1, **dadurch gekennzeichnet, dass** das Monomer a) mindestens eine Säuregruppe aufweist.

**3.** Verfahren gemäß Anspruch 2, **dadurch gekennzeichnet, dass** die Säuregruppen des Monomeren a) zumindest teilweise neutralisiert sind.

**4.** Verfahren gemäß einem der Ansprüche 1 bis 3, **dadurch gekennzeichnet, dass** Monomer a) zu mindestens 50 mol-% Acrylsäure ist.

**5.** Verfahren gemäß einem der Ansprüche 1 bis 4, **dadurch gekennzeichnet, dass** die Polymerpartikel einen mittleren Durchmesser von mindestens 200 $\mu$m aufweisen.

**6.** Verfahren gemäß einem der Ansprüche 1 bis 5, **dadurch gekennzeichnet, dass** mindestens 90 Gew.-% der Polymerpartikel einen Durchmesser von 100 bis 800 $\mu$m aufweisen.

**7.** Verfahren gemäß einem der Ansprüche 1 bis 6, **dadurch gekennzeichnet, dass** ein Trägergas durch den Reaktionsraum strömt.

**8.** Verfahren gemäß Anspruch 7, **dadurch gekennzeichnet, dass** das den Reaktionsraum verlassende Trägergas nach einem Durchgang zumindest teilweise rückgeführt wird.

**9.** Verfahren gemäß einem der Ansprüche 1 bis 8, **dadurch gekennzeichnet**, der Sauerstoffgehalt des Trägergases von 0,001 bis 0,15 Vol.-% beträgt.

**10.** Verfahren gemäß einem der Ansprüche 1 bis 9, **dadurch gekennzeichnet, dass** die erhaltenen Polymerpartikel in mindestens einem weiteren Verfahrensschritt getrocknet werden.

**11.** Wasserabsorbierende Polymerpartikel mit einer Zentrifugenretentionskapazität (CRC) von mindestens 20 g/g, einer mittleren Sphärizität von mindestens 0,84 und einer Absorption unter einem Druck von 4,83 kPa (AUL0.7psi), die die Bedingung

$$AUL0.7psi \geq -0{,}017\ CRC^2 + 1{,}5\ CRC - 6$$

erfüllt, wobei die Sphärizität (SPHT) definiert ist als

$$SPHT = \frac{4\pi A}{U^2},$$

wobei A die Querschnittsfläche und U der Querschnittsumfang der Polymerpartikel und die mittlere Sphärizität die volumengemittelte Sphärizität ist und die Zentrifugenretentionskapazität und die Absorption unter einem Druck von 4,83 kPa gemäß der von der EDANA empfohlenen Testmethoden bestimmt werden.

**12.** Polymerpartikel gemäß Anspruch 11, **dadurch gekennzeichnet, dass** die Polymerpartikel einen Gehalt an hydrophoben Lösungsmitteln von weniger als 0,005 Gew.-% aufweisen, wobei der Gehalt an hydrophobem Lösungsmittel gaschromatographisch mittels Head-Space-Technik bestimmt wird.

**13.** Polymerpartikel gemäß Anspruch 11 oder 12, **dadurch gekennzeichnet, dass** die Polymerpartikel eine Permeabilität von mindestens 2 x $10^{-7}$ $cm^3s/g$ aufweisen, wobei die Permeabilität gemäß der in der Beschreibung angegebenen Methode bestimmt wird.

**14.** Polymerpartikel gemäß einem der Ansprüche 11 bis 13, **dadurch gekennzeichnet, dass** die Polymerpartikel einen mittleren Durchmesser von mindestens 200 $\mu$m aufweisen, wobei der Partikeldurchmesser durch Lichtstreuung bestimmt wird und den volumengemittelten mittleren Durchmesser bedeutet.

**15.** Polymerpartikel gemäß einem der Ansprüche 11 bis 14, **dadurch gekennzeichnet, dass** mindestens 90 Gew.-% der Polymerpartikel einen Durchmesser von 100 bis 800 $\mu$m aufweisen.

**16.** Polymerpartikel gemäß einem der Ansprüche 11 bis 15, **dadurch gekennzeichnet, dass** die Polymerpartikel zumindest teilweise aus polymerisierten Säuregruppen tragenden, Monomeren a) bestehen.

**17.** Polymerpartikel gemäß Anspruch 16, **dadurch gekennzeichnet, dass** die Säuregruppen der polymerisierten Monomeren a) zumindest teilweise neutralisiert sind.

**18.** Polymerpartikel gemäß Anspruch 14 oder 17, **dadurch gekennzeichnet, dass** das Monomer a) zu mindestens 50 mol-% Acrylsäure ist.

**19.** Verwendung der Polymerpartikel gemäß einem der Ansprüche 11 bis 18 zur Herstellung von Hygieneartikeln.

**20.** Hygieneartikel, enthaltend Polymerpartikel gemäß einem der Ansprüche 11 bis 18.

**Claims**

**1.** A process for preparing water-absorbing polymer beads by polymerizing droplets of a monomer solution comprising

a) at least one ethylenically unsaturated monomer,

b) at least one crosslinker,
c) at least one initiator,
d) water,

in a gas phase surrounding the droplets, the resulting polymer beads being postcrosslinked, wherein the solids content of the monomer solution is at least 35% by weight and the polymer beads have a mean diameter of at least 150 μm, the solids content being the sum of monomer a), crosslinker b) and initiator c), the mean particle diameter being determined by light scattering and meaning the volume-average mean diameter.

**2.** The process according to claim 1, wherein the monomer a) has at least one acid group.

**3.** The process according to claim 2, wherein the acid groups of the monomer a) have beeen at least partly neutralized.

**4.** The process according to any of claims 1 to 3, wherein monomer a) is acrylic acid to an extent of at least 50 mol%.

**5.** The process according to any of claims 1 to 4, wherein the polymer beads have a mean diameter of at least 200 μm.

**6.** The process according to any of claims 1 to 5, wherein at least 90% by weight of the polymer beads have a diameter of from 100 to 800 μm.

**7.** The process according to any of claims 1 to 6, wherein a carrier gas flows through the reaction chamber.

**8.** The process according to claim 7, wherein the carrier gas leaving the reaction chamber is recycled at least partly after one pass.

**9.** The process according to any of claims 1 to 8, wherein the oxygen content of the carrier gas is from 0.001 to 0.15% by volume.

**10.** The process according to any of claims 1 to 9, wherein the resulting polymer beads are dried in at least one further process step.

**11.** Water-absorbing polymer beads having a centrifuge retention capacity (CRC) of at least 20 g/g, a mean sphericity of at least 0.84 and an absorbency under a load of 4.83 kPa (AUL0.7psi), which fulfills the condition that

$$\mathrm{AUL0.7psi} \geq -0.017\,\mathrm{CRC}^2 + 1.5\,\mathrm{CRC} - 6$$

where the sphericity (SPHT) is defined as

$$SPHT = \frac{4\pi A}{U^2},$$

where A is the cross-sectional area and U is the cross-sectional circumference of the polymer beads and the mean sphericity is the volume-average sphericity and the centrifuge retention capacity and the absorption under a pressure of 4.83 kPa are determined by the EDANA recommended test methods.

**12.** Polymer beads according to claim 11, which have a content of hydrophobic solvents of less than 0.005% by weight, the content of hydrophobic solvent being determined by gas chromatography by means of the headspace technique.

**13.** Polymer beads according to claim 11 or 12, which have a permeability of at least 2 x $10^{-1}$ $cm^3$ s/g, the permeability being determined by the method specified in the description.

**14.** Polymer beads according to any of claims 11 to 13, which have a mean diameter of at least 200 μm, the particle diameter being determined by light scattering and meaning the volume-average mean diameter.

**15.** Polymer beads according to any of claims 11 to 14, wherein at least 90% by weight of the polymer beads have a diameter of from 100 to 800 µm.

**16.** Polymer beads according to any of claims 11 to 15, which consist at least partly of polymerized acid-bearing monomers a).

**17.** Polymer beads according to claim 16, wherein the acid groups of the polymerized monomer a) have been at least partly neutralized.

**18.** Polymer beads according to claim 14 or 17, wherein monomer a) is acrylic acid to an extent of at least 50 mol%.

**19.** The use of the polymer beads according to any of claims 11 to 18 for producing hygiene articles.

**20.** A hygiene article comprising polymer beads according to any of claims 11 to 18.

**Revendications**

**1.** Procédé de fabrication de particules polymères absorbant l'eau par polymérisation de gouttes d'une solution de monomères, contenant :

a) au moins un monomère éthyléniquement insaturé,
b) au moins un agent de réticulation,
c) au moins un initiateur,
d) de l'eau,

dans une phase gazeuse enrobant les gouttes, les particules polymères obtenues étant post-réticulées, caractérisé ce ce que la teneur en solides de la solution de monomères est d'au moins 35 % en poids et les particules polymères présentent un diamètre moyen d'au moins 150 µm, la teneur en solides étant la somme du monomère a), de l'agent de réticulation b) et de l'initiateur c), le diamètre de particule moyen étant déterminé par diffusion de lumière et signifiant le diamètre moyen moyenné en volume.

**2.** Procédé selon la revendication 1, **caractérisé en ce que** le monomère a) comprend au moins un groupe acide.

**3.** Procédé selon la revendication 2, **caractérisé en ce que** les groupes acides du monomère a) sont au moins partiellement neutralisés.

**4.** Procédé selon l'une quelconque des revendications 1 à 3, **caractérisé en ce que** le monomère a) est l'acide acrylique à hauteur d'au moins 50 % en moles.

**5.** Procédé selon l'une quelconque des revendications 1 à 4, **caractérisé en ce que** les particules polymères présentent un diamètre moyen d'au moins 200 µm.

**6.** Procédé selon l'une quelconque des revendications 1 à 5, **caractérisé en ce qu'**au moins 90 % en poids des particules polymères présentent un diamètre de 100 à 800 µm.

**7.** Procédé selon l'une quelconque des revendications 1 à 6, **caractérisé en ce qu'**un gaz vecteur traverse la chambre de réaction.

**8.** Procédé selon la revendication 7, **caractérisé en ce que** le gaz vecteur quittant la chambre de réaction après un passage est au moins partiellement recyclé.

**9.** Procédé selon l'une quelconque des revendications 1 à 8, **caractérisé en ce que** la teneur en oxygène du gaz vecteur est de 0,001 à 0,15 % en volume.

**10.** Procédé selon l'une quelconque des revendications 1 à 9, **caractérisé en ce que** les particules polymères obtenues sont séchées lors d'au moins une étape de procédé supplémentaire.

**11.** Particules polymères absorbant l'eau ayant une capacité de rétention centrifuge (CRC) d'au moins 20 g/g, une sphéricité moyenne d'au moins 0,84 et une absorption sous une pression de 4,83 kPa (AUL 0,7 psi) qui satisfait la condition :

$$\text{AUL } 0{,}7 \text{ psi} \geq -0{,}017\ \text{CRC}^2 + 1{,}5\ \text{CRC} - 6$$

la sphéricité (SPHT) étant définie par

$$SPHT = \frac{4\pi A}{U^2},$$

A étant la surface de la section transversale et U la périphérie de la section transversale des particules polymères, et la sphéricité moyenne étant la sphéricité moyennée en volume, et la capacité de rétention centrifuge et l'absorption sous une pression de 4,83 kPa étant déterminées selon les méthodes d'essais recommandées par l'EDANA.

**12.** Particules polymères selon la revendication 11, **caractérisées en ce que** les particules polymères présentent une teneur en solvants hydrophobes de moins de 0,005 % en poids, la teneur en solvants hydrophobes étant déterminée par chromatographie gazeuse par la technique Headspace.

**13.** Particules polymères selon la revendication 11 ou 12, **caractérisées en ce que** les particules polymères présentent une perméabilité d'au moins 2 x $10^{-1}$ $cm^3s/g$, la perméabilité étant déterminée selon la méthode indiquée dans la description.

**14.** Particules polymères selon l'une quelconque des revendications 11 à 13, **caractérisées en ce que** les particules polymères présentent un diamètre moyen d'au moins 200 $\mu$m, le diamètre de particule étant déterminé par diffusion de lumière et signifiant le diamètre moyen moyenné en volume.

**15.** Particules polymères selon l'une quelconque des revendications 11 à 14, **caractérisées en ce qu'**au moins 90 % en poids des particules polymères présentent un diamètre de 100 à 800 $\mu$m.

**16.** Particules polymères selon l'une quelconque des revendications 11 à 15, **caractérisées en ce que** les particules polymères sont au moins partiellement constituées de monomères a) portant des groupes acides polymérisés.

**17.** Particules polymères selon la revendication 16, **caractérisées en ce que** les groupes acide des monomères a) polymérisés sont au moins partiellement neutralisés.

**18.** Particules polymères selon la revendication 14 ou 17, **caractérisées en ce que** le monomère a) est l'acide acrylique à hauteur d'au moins 50 % en moles.

**19.** Utilisation des particules polymères selon l'une quelconque des revendications 11 à 18 pour la fabrication d'articles d'hygiène.

**20.** Article d'hygiène, contenant des particules polymères selon l'une quelconque des revendications 11 à 18.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0348180 A
- WO 9640427 A
- US 5269980 A
- DE 10314466 A
- DE 10340253 A
- DE 102004024437 A
- DE 102005002412
- DE 102006001596
- DE 102004042946 A
- DE 102004042948 A
- DE 102004042955 A
- DE 102005019398
- WO 2005080479 A1
- DE 102004057868 A1
- DE 10239074 A1
- WO 2006042704 A1
- WO 2006079631 A1
- EP 0530438 A
- EP 0547847 A
- EP 0559476 A
- EP 0632068 A
- WO 9321237 A
- WO 03104299 A
- WO 03104300 A
- WO 03104301 A
- DE 10331450 A
- DE 10331456 A
- DE 10355401 A
- DE 19543368 A
- DE 19646484 A
- WO 9015830 A
- WO 0232962 A
- EP 0343427 A
- DE 4308842 A
- US 6338438 B
- EP 0083022 A
- EP 0543303 A
- EP 0937736 A
- DE 3314019 C
- DE 3523617 C
- EP 0450922 A
- DE 10204938 A
- US 6239230 B
- DE 4020780 A
- DE 198075022 A
- DE 19807992 A
- DE 198545732 A
- DE 10204937 A
- DE 10334584 A
- EP 1199327 A
- WO 03031482 A
- EP 0640330 A


### In der Beschreibung aufgeführte Nicht-Patentliteratur


- **F.L. BUCHHOLZ ; A.T. GRAHAM.** Modern Superabsorbent Polymer Technology. Wiley-VCH, 1998, 71-103